# EUROPEAN PATENT APPLICATION

(11) **EP 2 111 862 A1**
(43) Date of publication of application: **28.10.2009**
(21) Application number: 08702800.7
(22) Date of filing: 22.01.2008
(51) Int. Cl.: A61K 31/445, A61K 31/19, A61P 25/28, A61P 43/00

(54) **PHARMACEUTICAL PRODUCT FOR PREVENTION AND TREATMENT OF ALZHEIMER'S DEMENTIA**

(30) Priority: 23.01.2007 JP 2007012671
(71) Applicant: Nagoya City University, Aichi 467-8601 (JP); Kowa Company, Ltd., Naka-ku Nagoya-shi, Aichi-ken 460-8625 (JP)
(72) Inventor: OJIKA, Kosei, Nagoya-shi Aichi 467-8601 (JP); MATSUKAWA, Noriyuki, Nagoya-shi Aichi 467-8601 (JP)
(74) Representative: Polypatent
(86) International application number: PCT/JP2008/000059
(87) International publication number: WO 2008/090736

(57) **Abstract**

A medicament for prophylactic and/or therapeutic treatment of Alzheimer-type dementia, which comprises donepezil hydrochloride and valproic acid or a salt thereof in combination.

## Description

### Technical Field

The present invention relates to a medicament for prophylactic and/or therapeutic treatment of Alzheimer-type dementia. More specifically, the present invention relates to a medicament for prophylactic and/or therapeutic treatment of Alzheimer-type dementia, preferably a medicament for prophylactic and/or therapeutic treatment of behavioral and psychological symptoms of Alzheimer-type dementia, which comprises donepezil hydrochloride and valproic acid or a salt thereof in combination.

### Background Art

In recent years, in connection with rapid increase in population of aged persons, a problem of increasing patients with various senile mental disorders has been focused. In particular, senile dementia has potent influence not only on patients themselves, but on their surroundings such as families and communities, and therefore is becoming a social problem. Senile dementia is basically classified into Alzheimer-type dementia and cerebrovascular dementia caused by cerebrovascular attack. Among them, symptoms of Alzheimer-type dementia are roughly classified into cognitive symptoms such as ammesia, disorientation, and executive function disorder, and behavioral and psychological symptoms such as hallucination, delusion, depression, agitation, and wandering. Causes of Alzheimer-type dementia have not been revealed, and accordingly, no radical therapeutic method has yet been found.

Donepezil hydrochloride, a sole therapeutic drug for Alzheimer-type dementia approved in Japan, has been launched into market since July, 2004, and is considered to have certain effects for retarding onset or relieving of the cognitive symptoms. However, even some effects are not presently expected for the behavioral and psychological symptoms (Journal of the Japan Pharmaceutical Association, Vol. 58, No. 7, 861-864, 2006). Moreover, the improving action of donepezil hydrochloride against the cognitive symptoms is sustained for about one year at the longest, and thereafter the symptoms aggravate acceleratively, and various kinds of behavioral and psychological symptoms appear.

Further, it has recently been reported that new therapeutic drugs for schizophrenia used for suppressing paranoia and aggression of Alzheimer-type dementia cause side reactions similar to the symptoms of Parkinson's disease such as rigidity and dysbasia, besides aggravation of symptoms, weight increase and the like. Therefore, it is strongly desired to develop a novel therapeutic drug effective for Alzheimer-type dementia (N. Engl. J. Med., 355(15) 1525, 2006).

Sodium valproate acts on the cerebral center to suppress spasm and thereby suppress convulsive attack of epilepsy, and therefore has been used as an antiepileptic for many years. Usefulness of sodium valproate for prophylactic or therapeutic treatment of neurodegenerative diseases has been reported as applications thereof as an astrocytic function improving agent, reactive astrocytic induction inhibitor, and agent for conversion of reactive astrocyte into astrocyte containing sodium valproate. However, therapeutic effect on Alzheimer-type dementia has not been specifically described (Japanese Patent Unexamined Publication (KOKAI) No. 10-324626).

It has been reported that a clinical test was performed in the United States in which sodium valproate was solely administered to Alzheimer-type dementia patients, and that no improving effect on the behavioral and psychological symptoms such as agitation was observed (Am. J. Geriatr. Psychiatry, 13(11) 942, 2005).
Non-patent document 1: Journal of the Japan Pharmaceutical Association, Vol. 58, No. 7, 861-864, 2006
Non-patent document 2: Am. J. Geriatr. Psychiatry, 13(11) 942, 2005
Non-patent document 3: N. Engl. J. Med., 355(15) 1525, 2006
Patent document 1: Japanese Patent Unexamined Publication (KOKAI) No. 10-324626

### Disclosure of the Invention

### Object to be Achieved by the Invention

An object of the present invention is to provide a medicament for treatment of Alzheimer-type dementia.
More specifically, the object of the present invention is to provide a medicament for treatment of Alzheimer-type dementia, preferably a medicament for treatment of behavioral and psychological symptoms accompanying Alzheimer-type dementia, more preferably a medicament having an action for prophylactic and/or therapeutic treatment of the behavioral and psychological symptoms accompanied by problem behaviors.

### Means for Achieving the Object

In order to achieve the aforementioned object, the inventors of the present invention conducted various researches, and by using combinations of donepezil hydrochloride and variety of drugs, they examined prolonging effect on the therapeutic effect of donepezil hydrochloride on the cognitive symptoms of Alzheimer-type dementia, and prophylactic and/or therapeutic effect on the behavioral and psychological symptoms of the Alzheimer-type dementia, especially the behavioral and psychological symptoms accompanied by problem behaviors. As a result, when valproic acid or a salt thereof and donepezil hydrochloride were administered in combination, the therapeutic effect of donepezil hydrochloride on the cognitive symptoms was found to be continued for a longer period of time as compared with that obtainable by sole administration of donepezil hydrochloride. Further, although the behavioral and psychological symptoms of dementia become not suppressible with donepezil hydrochloride when the symptoms of dementia advanced, when donepezil hydrochloride and valproic acid were administered in combination, it was found that the onset of the behavioral and psychological symptoms was significantly suppressed and the behavioral and psychological symptoms were improved. The present invention was accomplished on the basis of the aforementioned findings.

The present invention thus provides a medicament for prophylactic and/or therapeutic treatment of Alzheimer-type dementia, which comprises donepezil hydrochloride and valproic acid or a salt thereof in combination.
According to preferred embodiments of the present invention, there are provided the aforementioned medicament, which is in the form of a pharmaceutical composition comprising donepezil hydrochloride and valproic acid or a salt thereof, and the aforementioned medicament, which comprises donepezil hydrochloride and valproic acid or a salt thereof in combination in a form for administering donepezil hydrochloride and valproic acid or a salt thereof simultaneously, separately, or successively.

From other aspects, the present invention provides use of donepezil hydrochloride for manufacture of the aforementioned medicament, use of valproic acid or a salt thereof for manufacture of the aforementioned medicament, use of donepezil hydrochloride and valproic acid or a salt thereof for manufacture of the aforementioned medicament, and a method for prophylactic and/or therapeutic treatment of Alzheimer-type dementia, which comprises the step of administering a combination of donepezil hydrochloride and valproic acid or a salt thereof, simultaneously, separately, or successively.
From another aspect, the present invention provides a medicament for prolonging therapeutic period of donepezil hydrochloride effective on the cognitive symptoms, which comprises valproic acid or a salt thereof as an active ingredient.
The present invention also provides a medicament for prophylactic and/or therapeutic treatment of behavioral and psychological symptoms of Alzheimer-type dementia occurring during treatment of Alzheimer-type dementia with donepezil hydrochloride, which comprises valproic acid or a salt thereof as an active ingredient.

### Effect of the Invention

The medicament of the present invention has significantly prolonged therapeutic period effective on the cognitive symptoms of Alzheimer-type dementia as compared with that obtainable with donepezil hydrochloride, which is conventionally used as a therapeutic drug for Alzheimer-type dementia. The medicament can effectively prevent the onset of behavioral and psychological symptoms of Alzheimer-type dementia, which cannot be avoided with the sole administration of donepezil hydrochloride, and the medicament can achieve effective therapeutic effects such as suppression and improvement also on behavioral and psychological symptoms after the onset. Since valproic acid alone does not exhibit any improving effect on the behavioral and psychological symptoms of Alzheimer-type dementia patients (Am. J. Geriatr. Psychiatry, 13(11) 942, 2005), the combinatorial effect of the aforementioned active ingredients of the medicament of the present invention is very surprising. Since valproic acid and a salt thereof have little side reactions such as sleepiness, the medicament of the present invention is useful as a medicament for prophylactic and/or therapeutic treatment of Alzheimer-type dementia.

### Best Mode for Carrying out the Invention

Donepezil hydrochloride has been clinically used as a medicament for Alzheimer-type dementia for suppressing progress of demential symptoms in mild or moderate Alzheimer-type dementia ("Aricept", produced and distributed by Eisai Co., Ltd.), and can be easily obtained.
Valproic acid or a salt thereof is used for therapeutic treatment of various kinds of epilepsy (minor epilepsy, focal seizure, psychomotor seizure, and mixed seizure), therapeutic treatment of character behavior disorders accompanying epilepsy (morose, trait anger and the like), and therapeutic treatment of mania and manic state in manic-depressive psychosis, and is readily available. As valproic acid or a salt thereof, for example, sodium valproate can be preferably used.

The medicament of the present invention is characterized to comprise donepezil hydrochloride and valproic acid or a salt thereof in combination. Although form of the medicament of the present invention is not particularly limited, the form may be, for example, a pharmaceutical composition comprising donepezil hydrochloride and valproic acid or a salt thereof (i.e., a formulation containing a mixture), or a medicament consisting of a combination of separate administration units of donepezil hydrochloride and valproic acid or a salt thereof for separately, separately, or successively administering donepezil hydrochloride and valproic acid or a salt thereof. For example, tablets or subtilized granules containing donepezil hydrochloride as an active ingredient as well as tablets, syrups, sustained release granules, subtilized granules and the like containing sodium valproate as an active ingredient are clinically used, and therefore the medicament of the present invention may be prepared by using these commercially available preparations per se are as a medicament consisting of a suitable combination thereof.

Although amounts and combination ratios of the aforementioned two kinds of components in the medicament of the present invention are not particularly limited, they are preferably combined by referring to clinical doses of donepezil hydrochloride and valproic acid or a salt thereof. For example, they can be combined so that a daily dose of donepezil hydrochloride can be about 1 to 10 mg, preferably about 3 to 5 mg, and a daily dose of valproic acid or a salt thereof can be about 100 to 2,000 mg, preferably 400 to 1,200 mg. Administration of donepezil hydrochloride may be started with a daily dose of 3 mg, and the dose may be increased to about 5 mg after one to two weeks. As for donepezil hydrochloride, the administration method including the increase dose is preferred. Administration method of the medicament of the present invention is not particularly limited, and for example, an administration method of simultaneously or separately administering donepezil hydrochloride and valproic acid or a salt thereof, or successively administering said substance with an interval of several hours to several days can be exemplified. When they are successively administered, either of the active ingredients may be administered first. Although it is preferable to administer donepezil hydrochloride once a day and valproic acid or a salt thereof several times a day (2 to 3 times in the case of tablets, once or twice in the case of sustained release tablets, and about once in the case of sustained release granules), administration method is not particularly limited.

The medicament of the present invention can prolong the effective therapeutic period of donepezil hydrochloride against the cognitive symptoms as compared with that obtainable solely with donepezil hydrochloride, which is conventionally used as a therapeutic agent of Alzheimer-type dementia. The effectiveness of donepezil hydrochloride against the cognitive symptoms is usually eliminated or reduced after about one year, whist effectiveness of the medicament of the present invention against the onset and advance of the cognitive symptoms can be expected for at least one year or more, preferably several years or more. Further, the medicament of the present invention can effectively prevent the onset of the behavioral and psychological symptoms, preferably behavioral and psychological symptoms accompanied by problem behaviors, which cannot be avoided with sole administration of donepezil hydrochloride, or effective therapeutic effects such as suppression action and amelioration action on the behavioral and psychological symptoms after the onset thereof can be expected.

Furthermore, a medicament comprising valproic acid or a salt thereof as an active ingredient can be used as a medicament for prolonging therapeutic period of donepezil hydrochloride effective against the cognitive symptoms, or a medicament for prophylactic and/or therapeutic treatment of behavioral and psychological symptoms of Alzheimer-type dementia occurring during therapeutic treatment of Alzheimer-type dementia with donepezil hydrochloride. These medicaments also fall within the scope of the present invention.

### Examples

The present invention will be more specifically explained with reference to examples. However, the scope of the present invention is not limited by the following examples.

### Example 1

Female patient. After her relative passed away six years ago, amnesia became conspicuous, and she became unable to appropriately do housekeeping about five years ago (53 years old at that time). She was diagnosed as Alzheimer-type dementia after three years from then at the age of 56 (two years ago), and administration of donepezil hydrochloride chewable tablets (once a day after breakfast at a dose of 5 mg) was started two months afterward. Even after two months from the start of the administration, actions of making an emergency call and the like were observed, and problem behaviors did not subside. After 11 months from the start of the donepezil hydrochloride administration, concomitant administration of sustained release tablets of sodium valproate (twice a day after breakfast and dinner with 200 mg tablet x 2) was started. Agitation decreased from two months after the start of the concomitant administration of sustained release tablets of sodium valproate, and agitation symptoms disappeared in eight months after the start of the administration of the sustained release tablets of sodium valproate.

### Example 2

Male patient. Amnesia was observed from six years ago (55 years old at that time), and he retired two years afterward (four years ago), as he became unable to maintain good personal relations at work, and diagnosed as Alzheimer-type dementia on the same year (four years ago) at the age of 57. Although he showed symptoms of short term memory obstruction, disorientation for time, day of the week, season, topography and the like, acalculia and the like, he had no fugue and could make easy judgments for daily living. Thereafter, dementia advanced, agnosia and apraxia were observed, and administration of donepezil hydrochloride (once a day after breakfast at a dose of 5 mg) was started six months after the diagnosis. Even two months after the start of the donepezil hydrochloride administration, symptoms of incontinentia and the like were observed, and even four months after the start of the administration, visual hallucination, incontinentia, aggressive attitude and the like were observed. After 35 months from the start of the donepezil hydrochloride administration, concomitant administration of sustained release tablets of sodium valproate (twice a day after breakfast and dinner with 200 mg tablet x 2) was started. Visual hallucination was observed even one month after the start of the sodium valproate administration, and thereafter sodium valproate was administered twice a day after breakfast and dinner with 200 mg tablet x 4. From 3 months after the start of the sodium valproate administration, drowse was more often observed, and five months afterward, he became quieter than before, but visual hallucination and incontinentia were observed. Seven months after the start of the sodium valproate administration, visual hallucination decreased, and violent behaviors were also disappeared, but behavior disorder due to delusion remained. However, rioting at night disappeared.

### Example 3

Female patient. She complained dysgraphia six years ago (76 years old at that time), and acalculia at the time of shopping five years ago, and she became unable to write even her own name from four years ago. Three years ago, she was diagnosed as Alzheimer-type dementia at the age of 79, but she showed little visual hallucination and pseudopsia. Although administration of donepezil hydrochloride (once a day after breakfast at a dose of 5 mg) was started seven months after the diagnosis, dressing apraxia was observed 15 months after the start of the administration, and visual hallucination, soliloquy, emotional attitude, dressing apraxia, and stereotypy were observed 23 months after the start of the donepezil hydrochloride administration. Even 25 months after the start of the donepezil hydrochloride administration, she showed visual hallucination, delusion of persecution and the like, and concomitant administration of sustained release tablets of sodium valproate (twice a day after breakfast and dinner with 200 mg tablet x 3) was started on the same month. Even one month after the start of the sodium valproate administration, symptoms of delusion of persecution, visual hallucination, night wandering and the like were observed, but she came to take sleep at night five months after the start of the concomitant administration. Seven months after the start of the sodium valproate administration, she became calm, and agitation also disappeared.

### Example 4

Female patient. Her memory was clouded from about two years ago (78 years old at that time), and she sometimes was unable to write her own name and unable to have good relation with her family. One year ago (79 years old at that time), she had a medical examination, in which dysmnesia, disorientation, and acalculia were observed, and diagnosed to be suspected Alzheimer-type dementia. Two months after the diagnosis, she suffered from visual hallucination, and administration of donepezil hydrochloride chewable tablets (once a day after breakfast at a dose of 5 mg) was started on the same month. Two months after the start of the donepezil hydrochloride administration, visual hallucination was not observed, but she spoke only her events in the old days and showed stereotypy, and therefore concomitant administration of sustained release tablets of sodium valproate (twice a day after breakfast and dinner with 200 mg tablet x 2) was started on the same month. One month after the start of the sodium valproate administration, the condition did not change, and she repeatedly spoke the same thing and was emotional and likely to get angry, but she became able to take sleep at night. Three months after the start of the sodium valproate administration, she did not suffer from visual hallucination, and she became able to do face washing, brushing her teeth, choosing and putting her clothes, and the like by herself. Seven months after the start of the administration, although symptoms of making noise intermittently, forgetting rapidly and the like remained, she showed no other special change.

### Example 5

Female patient. She got insomnia ten years ago (56 years old at that time) due to troubles in her life, and came to suffer from amnesia from eight years ago (58 years old at that time). Amnesia gradually got aggravated, then she visited the clinic five years ago, and was diagnosed as Alzheimer-type dementia (61 years old at that time), and administration of donepezil hydrochloride (once a day after breakfast at a dose of 5 mg) was started. In the inspection 24 months after the start of the donepezil hydrochloride administration, atrophy of the hippocampus was marked, and cerebral blood flow was also falling extensively, but her topographical orientation was maintained, and she could chose and put away and out her clothing, and do shopping in the neighborhood. However, orientation for season and date or calculation was disturbed, and refusal of bathing and the like was increasing. Thirty nine months after the start of the donepezil hydrochloride administration, cognitive disorder advanced, and 41 months after the start of the donepezil administration, she fell down from the step at night, and showed refusal of oral administration of medicaments, soliloquy and dressing apraxia. Thereafter, she also showed excretion action in the veranda and the like at night. Concomitant administration of sustained release tablets of sodium valproate (twice a day after breakfast and dinner with 200 mg tablet x 2) was started 48 months after the start of the donepezil hydrochloride administration. Two months after the start of the concomitant administration, symptoms were ameliorated, and she no longer showed striking walls or fugue, and also came to sleep at night. Four months after the start of the concomitant administration, she stopped getting angry, and after eight months, she could sleep at night, did not show wandering and less frequently made noise, although she suffered from incontinentia and the like Thus, peripheral symptoms were improved. Sustained release tablets of sodium valproate (200 mg tablet x 3) were administered from eight months after the start of the concomitant administration. Few secondary obstructions were observed, and her life was considerably calmed down, for example, she got along with her family at home.

The results of Examples 1 to 5 and improvement degrees of the behavioral and psychological symptoms are summarized as shown in Table 1. As specifically demonstrated in the aforementioned cases, the combination of donepezil hydrochloride and valproic acid or a salt thereof can effectively suppress advance of cognitive symptoms of Alzheimer-type dementia, delay expression of behavioral and psychological symptoms thereof, and improve the behavioral and psychological symptoms. Therefore, the medicament of the present invention can be used as an extremely useful medicament for prophylactic and/or therapeutic treatment of Alzheimer-type dementia under the current circumstance that no fundamental therapy has been established.

**[Table 1]**

| Case No. | Sex Age at onset Age at diagnosis | Administration period of donepezil alone before start of concomitant administration (month) | Pathological phase ofAD at start of concomitant administration *1 | Dose of VPA after start of concomitant administration (mg/twice/day) | Time of medical examination after start of concomitant administration (month) | Improvement factor of peripheral symptoms *2 |
|---|---|---|---|---|---|---|
| 1 | Female 53 years old | 11 | Late I | 400 | 2 | 4 |
| | 56 years old | | | | | |
| 2 | Male | 35 | II | 400 | 1 | 2 |
| | 55 years old | | | 800 (from 1 month after) | 5 | 3 |
| | 57 years old | | | | 7 | 4 |
| 3 | Female | 25 | II | 600 | 1 | 2 |
| | 76 years old | | | | 5 | 3 |
| | 79 years old | | | | 7 | 4 |
| 4 | Female | 2 | Early II | 400 | 1 | 2 |
| | 78 years oId | | | | 3 | 4 |
| | 79 years old | | | | 7 | 4 |
| 5 | Female | 48 | Early II | 400 | 2 | 3 |
| | 58 years old | | | 600 (from 8 months after) | 4 | 3 |
| | 61 years old | | | | 8 | 4 |

| | | | | | | |
|---|---|---|---|---|---|---|
| *1: Sjögren classification (I: mild peripheral symptoms, II: advanced peripheral symptoms) *2: 1: Aggravation, 2: no change, 3: slight improvement, 4: improvement | | | | | | |

### Industrial Applicability

The medicament of the present invention containing donepezil hydrochloride and valproic acid or a salt thereof in combination is useful as a medicament for prophylactic and/or therapeutic treatment of Alzheimer-type dementia.

## Claims

1. A medicament for prophylactic and/or therapeutic treatment of Alzheimer-type dementia, which comprises donepezil hydrochloride and valproic acid or a salt thereof in combination.

2. The medicament according to claim 1, which is in the form of a pharmaceutical composition comprising donepezil hydrochloride and valproic acid or a salt thereof.

3. The medicament according to claim 1, which comprises donepezil hydrochloride and valproic acid or a salt thereof in combination in a form for administering donepezil hydrochloride and valproic acid or a salt thereof simultaneously, separately or successively.

4. The medicament according to any one of claims 1 to 3, which is used for prophylactic and/or therapeutic treatment of behavioral and psychological symptoms of Alzheimer-type dementia.

5. The medicament according to claim 4, which is used for prophylactic and/or therapeutic treatment of the behavioral and psychological symptoms accompanied by problem behaviors.

6. A medicament for prolonging effective therapeutic period of donepezil hydrochloride for cognitive symptoms, which comprises valproic acid or a salt thereof as an active ingredient.

7. A medicament for prophylactic and/or therapeutic treatment of behavioral and psychological symptoms ofAlzheimer-type dementia occurring during therapeutic treatment ofAlzheimer-type dementia with donepezil hydrochloride, which comprises valproic acid or a salt thereof as an active ingredient.
